# FASCICULE DE BREVET EUROPEEN

(11) **EP 3 321 369 B1**
(45) Date de publication et mention de la délivrance du brevet: **03.04.2019**
(21) Numéro de dépôt: 17290150.6
(22) Date de dépôt: 15.11.2017
(51) Int. Cl.: C12Q 1/02, A61L 2/28, C12M 1/34, C12Q 1/22, G01N 35/00

(54) **MÉTHODE DE VALIDATION D'UN PROCÉDÉ DE STÉRILISATION COMPORTANT DEUX CONTAMINATIONS SUCCESSIVES**
VALIDIERUNGSMETHODE EINES STERILISATIONSVERFAHRENS, DAS ZWEI AUFEINANDERFOLGENDE KONTAMINATIONEN BEINHALTET
METHOD FOR VALIDATING A STERILISATION PROCESS COMPRISING TWO SUCCESSIVE CONTAMINATIONS

(30) Priorité: 15.11.2016 FR 1601614
(43) Date de publication de la demande: 16.05.2018
(73) Titulaire: Biron, Jean-François, 33700 Merignac (FR)
(72) Inventeur: Biron, Jean-François, 33700 Merignac (FR)
(74) Mandataire: Degret, Jacques

(56) Documents cités:
- WO-A1-2014/159696
- US-A1- 2008 095 676
- US-A1- 2015 147 773
- US-B1- 6 428 746
- US-B1- 8 808 620
- N/a: "Sterilization of health care products - Moist heat - Part 1: Requirements for the development, validation and routine control of a sterilization process for medical devices", National Standards Authority of Ireland, EN ISO 17665-1: 2006, 1 janvier 2006 (2006-01-01), pages 1-38, XP055306730, Extrait de l'Internet: URL:https://www.iso.org/standard/43187.htm l [extrait le 2016-09-29]
- N/a: "European Pharmacopoeia 5.0", , 1 janvier 2005 (2005-01-01), XP002772264, Extrait de l'Internet: URL:http://library.njucm.edu.cn/yaodian/ep /EP5.0/index.html [extrait le 2017-07-17]
- B M Boca ET AL: "An Overview of the Validation Approach for Moist Heat Sterilization, Part I", Pharmaceutical Technology, 1 septembre 2002 (2002-09-01), XP055300049, Extrait de l'Internet: URL:http://www.gmpua.com/Equipment/Sterili ty/Sterilization/article.pdf [extrait le 2016-09-06]
- SYLVIE GUYOMARD: "Suivi de la biocharge (Bioburden) des solutions stériles avant stérilisation en 2010", La Vague LA VAGUE, vol. 30 1 septembre 2010 (2010-09-01), pages 16-18, XP002772145, ISSN: 1298-0471 Extrait de l'Internet: URL:http://a3p.org/images/stories/article/ scientifique/vague30/0pdf_articles/30pdf7. pdf [extrait le 2017-07-17]

## Description

### DOMAINE TECHNIQUE DE L'INVENTION

La présente invention concerne une méthode de validation d'un procédé de stérilisation d'un produit, notamment d'un produit de santé (médicament, dispositif médical, produit cosmétique, produit biotechnologique, etc....) conditionné dans un contenant qui peut être ouvert et rescellé ensuite.

### ARRIÈRE-PLAN TECHNOLOGIQUE DE L'INVENTION

L'état de stérilité de certains articles et plus particulièrement l'état de stérilité d'articles pour la santé, notamment les produits injectables ainsi que les contenants de ces produits, est un souci constant pour l'homme de l'art comme pour les autorités de santé. L'état de stérilité est défini dans différentes normes et textes réglementaires sous la forme d'un niveau d'assurance de stérilité, désigné par l'acronyme « NAS », qui est la probabilité de non stérilité de chaque article - produit ou contenant - stérilisé.

Le niveau d'assurance de stérilité NAS retenu impose généralement une concentration statistique inférieure à un seuil de 10⁻⁶ unités formant colonies vivantes, désignées par l'acronyme « UFC », ce qui correspond à une cellule vivante de micro-organisme au maximum pour un million d'articles, soit encore une probabilité de non stérilité d'une « chance » sur un million pour chaque article stérilisé.

Les procédés de stérilisation peuvent être classés en deux catégories en fonction des courbes obtenues :
a) une première catégorie de procédés de stérilisation pour lesquels, suivant certains auteurs et un certain nombre de textes réglementaires, il est possible de déduire une courbe de décroissance exponentielle du nombre de cellules vivantes de micro-organisme, en fonction d'un paramètre tel que le temps d'exposition ou une dose d'irradiation appliquée ; ces procédés consistent notamment en la stérilisation par chaleur humide, par chaleur sèche, par l'oxyde d'éthylène, ainsi qu'en la stérilisation par irradiation bêta ou gamma,
b) une seconde catégorie de procédés de stérilisation pour lesquels il n'est pas possible de déduire une courbe de décroissance exponentielle du nombre de cellules vivantes de micro-organismes en fonction d'un paramètre tel que le temps d'exposition ou un dosage appliqué, laquelle seconde catégorie comprend notamment la stérilisation par le peroxyde d'hydrogène, par l'acide peracétique, ou encore par hyperpression hydrostatique.

Une première méthode permettant d'évaluer le niveau d'assurance de stérilité NAS pour les procédés de stérilisation de la première catégorie utilise des indicateurs biologiques préparés avec le micro-organisme défini ou, si possible, prouvé comme étant le plus difficile à détruire avec le procédé de stérilisation choisi ; ces indicateurs biologiques sont inoculés dans des échantillons de l'article - produit ou contenant - à stériliser. Après la stérilisation, on mesure le nombre de cellules vivantes de micro-organismes restantes en faisant évoluer certains paramètres du procédé de stérilisation, le plus souvent le temps de stérilisation ou la dose, et ce afin de pouvoir évaluer les interactions entre l'article et ce procédé.

On obtient par cette méthode une courbe de décroissance du nombre de cellules vivantes de micro-organismes en fonction de plusieurs paramètres de stérilisation, comprenant généralement le temps d'exposition pour les procédés de stérilisation utilisant la chaleur humide, ou la chaleur sèche, ou l'oxyde d'éthylène, et comprenant la dose reçue pour les procédés de stérilisation par irradiation bêta ou gamma.

Pour cette première méthode d'évaluation, on prolonge généralement la courbe pour les très faibles niveaux de contamination, comportant statistiquement moins d'une seule cellule de micro-organisme survivante, afin d'en déduire les caractéristiques du procédé de stérilisation permettant d'atteindre le niveau d'assurance de stérilité NAS recherché.

Cette méthode pose toutefois un problème car l'extrapolation de la courbe pour les très faibles niveaux de contamination repose sur une hypothèse que l'expérience ne peut pas toujours prouver. Certaines normes imposent d'atteindre un coefficient minimum de corrélation pour des valeurs élevées de contamination par des mesures qui sont en effet difficiles à effectuer.

Une deuxième méthode permettant d'évaluer le niveau d'assurance de stérilité NAS pour les procédés de stérilisation de la seconde catégorie prévoit l'inoculation des articles à stériliser avec des indicateurs biologiques présentant un nombre voulu de cellules vivantes du micro-organisme, souvent compris entre 10³ et 10⁶ cellules, puis, après le traitement de stérilisation, le contrôle de la destruction de l'ensemble des cellules vivantes de ces indicateurs.

Pour cette méthode, une courbe de décroissance du nombre de cellules vivantes de micro-organismes en fonction d'un paramètre, tel le temps d'exposition ou une dose de stérilisation appliquée ou encore le nombre de cellules inoculées, ne peut pas être calculée. La probabilité de non stérilité ne peut donc pas être évaluée non plus, ce qui pose des problèmes de garantie du NAS. Dans certains cas, tant la réglementation que l'homme de l'art considèrent alors qu'il s'agit d'une décontamination sans obligation d'atteinte de l'état de stérilité.

### DESCRIPTION GÉNÉRALE DE L'INVENTION

La présente invention a notamment pour but d'éviter tous les problèmes de l'art antérieur exposés ci-dessus.

Elle propose à cet effet une méthode de validation d'un procédé de stérilisation d'un article quelconque, par exemple un contenant ou un produit contenu dans un contenant adapté pour le procédé de stérilisation considéré. Cette méthode est applicable à tous les procédés de stérilisation et elle vise principalement mais non limitativement les produits et les dispositifs destinés à la santé.

Le brevet US - 6.428.746 publié le 6 août 2002 décrit une méthode de validation d'un procédé de stérilisation d'un article, notamment un produit destiné à la santé, permettant de valider le Niveau d'Assurance de Stérilité atteint avec ce procédé de stérilisation, cette méthode consistant à réaliser une et une seule étape de contamination de l'article par plus de 10⁵ cellules vivantes de micro-organismes, à réaliser ensuite un cycle de stérilisation avec le procédé retenu et enfin à vérifier la stérilité de l'article après le cycle de stérilisation.

Conformément à l'invention, la méthode de validation d'un procédé de stérilisation d'un article quelconque, permettant de valider le niveau d'assurance de stérilité atteint avec ce procédé de stérilisation, se caractérise par le fait qu'elle consiste à réaliser une première étape de contamination d'un contenant recevant l'article par plus de 10⁵ cellules vivantes de micro-organisme, à réaliser ensuite un premier cycle de stérilisation avec le procédé de stérilisation retenu, à ouvrir ensuite le contenant pour le contaminer à nouveau par plus de 10⁵ cellules vivantes de micro-organisme, puis à réaliser un deuxième cycle de stérilisation avec le même procédé, et enfin à vérifier la stérilité du contenant après le premier cycle et après le deuxième cycle de stérilisation.

La méthode de validation selon l'invention peut de plus comporter une ou plusieurs des caractéristiques suivantes, qui peuvent être combinées entre elles.

Ainsi, avantageusement, chaque contamination est effectuée avec au moins 10⁶ cellules vivantes de micro-organisme.

Avantageusement, les contaminations sont faites avec les cellules vivantes du micro-organisme prouvé comme étant le plus difficile à éliminer avec le procédé de stérilisation retenu.

Avantageusement, la vérification de la stérilité du contenant comporte la révélation d'indicateurs biologiques démontrant qu'aucun de ces indicateurs n'a réalisé une croissance.

Avantageusement, les deux contaminations sont effectuées dans les mêmes conditions, et les deux cycles de stérilisation sont également effectués dans les mêmes conditions, sauf nécessité due au procédé et notamment si le deuxième cycle ne permet pas de détruire au moins 5 logs de cellules vivantes du micro-organisme le plus difficile à détruire par le procédé de stérilisation considéré.

Avantageusement, la méthode de validation selon l'invention permet de modifier le deuxième cycle de stérilisation pour s'assurer de l'atteinte réelle de la destruction d'au moins 5 logs de cellules vivantes du micro-organisme le plus difficile à détruire par le procédé de stérilisation considéré.

Avantageusement, l'invention permet une réalisation des opérations d'ouverture, de re-contamination, et de fermeture des contenants très rapidement afin d'optimiser le temps de réalisation des cycles de routine, composés du premier cycle, d'un temps d'attente égal à ces phases d'ouverture, de re-contamination et de fermeture de ces contenants dans les mêmes conditions environnementales, et du deuxième cycle.

L'objectif de la présente méthode est de prouver un Niveau d'Assurance de Stérilité (NAS) d'au moins 10⁻⁵ pour une contamination initiale de 10⁵ cellules vivantes du micro-organisme prouvé comme étant le plus difficile à détruire par le procédé de stérilisation considéré. Cette méthode est totalement indépendante du mode d'action du procédé de stérilisation, et notamment du fait que le procédé de stérilisation appartienne à la première ou à la seconde catégorie citée ci-dessus.

L'obtention d'un tel NAS repose sur l'obtention d'une réduction de contamination d'au moins 10 logs.

La présente méthode est remarquable en ce qu'elle prouve réellement ces 10 logs de réduction de contamination, ce qu'aucune méthode ne permet d'atteindre aujourd'hui. Elle consiste à inoculer l'article à stériliser avec au moins 10⁵ cellules vivantes du micro-organisme avantageusement prouvé comme étant le plus difficile à détruire par le procédé de stérilisation considéré, à réaliser un premier cycle de stérilisation qui parvienne à détruire ces au moins 10⁵ cellules vivantes de ce micro-organisme, puis à ouvrir les contenants, à les ré-inoculer, et à les refermer avec à nouveau au moins 10⁵ cellules vivantes du micro-organisme prouvé comme étant le plus difficile à détruire par le procédé de stérilisation considéré, puis à réaliser un deuxième cycle de stérilisation identique au premier, qui parvienne à détruire à nouveau au moins 10⁵ cellules vivantes du micro-organisme le plus difficile à détruire par ce procédé de stérilisation.

Les opérations d'ouverture des contenants, de ré-inoculation, et de re-fermeture sont réalisés au moyen d'un des dispositifs conformer à l'invention.

A l'issue du premier cycle de stérilisation, des échantillons contaminés avec 10⁵ UFC du micro-organisme le plus difficile à détruire par le procédé de stérilisation considéré sont prélevés et soumis à un test connu de l'homme de l'art et/ou décrit dans les textes réglementaires pour permettre de prouver la destruction de ces au moins 10⁵ cellules vivantes du micro-organisme. Il est donc prouvé à l'issue de ce premier cycle que ce dernier est capable de détruire 5 logs de contamination.

A l'issue du deuxième cycle de stérilisation, des échantillons contaminés avec 10⁵ cellules vivantes du micro-organisme le plus difficile à détruire par le procédé de stérilisation considéré sont également prélevés et soumis au même test connu de l'homme de l'art et/ou décrit dans les textes réglementaires pour permettre de prouver la destruction de ces au moins 10⁵ cellules vivantes du micro-organisme le plus difficile à détruire par le procédé de stérilisation considéré.

Il est donc prouvé à l'issue de ce deuxième cycle que ce dernier est capable de détruire au moins 5 logs supplémentaires de cellules vivantes du micro-organisme cité ci-dessus.

La réalisation de ce deuxième cycle est indispensable, car le contenant et le produit ou l'article qu'il enferme ont déjà subi un cycle de stérilisation, lequel cycle peut avoir affecté leurs caractéristiques et leur comportement lors d'un nouveau cycle de stérilisation. En l'absence de test, il n'est pas possible d'affirmer que le deuxième cycle identique au premier permet réellement de détruire au moins 5 logs supplémentaires de cellules vivantes du micro-organisme le plus difficile à détruire par le micro-organisme considéré.

Dans le cas où le deuxième cycle, pour les raisons évoquées ci-dessus, ne permettrait pas la réduction d'au moins 5 logs supplémentaires de cellules vivantes de ce micro-organisme, l'homme de l'art pourra très bien modifier les caractéristiques de ce deuxième cycle pour atteindre cette réduction d'au moins 5 logs.

La présente méthode est en ceci remarquable qu'elle permet de prouver la destruction d'au moins 5 logs pour le premier cycle et d'au moins 5 logs supplémentaires pour le deuxième cycle, ce qu'aucune méthode ne permet de réaliser à ce jour.

La présente méthode est en ceci remarquable qu'elle permet de démontrer que le cycle de routine composé du premier cycle, d'un temps d'attente dans les mêmes conditions que celles expérimentées pour ouvrir les contenants, les ré-inoculer, et les refermer selon l'invention, puis d'un deuxième cycle, permet de détruire au moins 10 logs du micro-organisme le plus difficile à détruire par le procédé de stérilisation considéré.

Comme démontré plus haut, un NAS d'au moins 10⁻⁵ est atteint si la contamination initiale avant stérilisation des contenants à stériliser est inférieure ou égale à 10⁵ UFC du micro-organisme le plus difficile à détruire par le procédé de stérilisation considéré, et que le procédé de stérilisation permet de détruire au moins 10 logs de ce micro-organisme.

La présente méthode est en ceci remarquable qu'elle permet de démontrer ce NAS d'au moins 10⁻⁵ si la contamination initiale est inférieure à 10⁵ cellules du micro-organisme le plus difficile à détruire par le procédé de stérilisation étudié.

Une installation prévue pour mettre en oeuvre une telle méthode de validation est décrite. Elle comprend l'une quelconque des caractéristiques précédentes, laquelle installation comporte des moyens permettant le déplacement successif des contenants sur un poste de travail comprenant au moins un système d'ouverture, des moyens d'inoculation et au moins un système de fermeture de ces contenants.

Selon un premier mode de réalisation de cette installation, le au moins un système d'ouverture, les moyens d'inoculation et le au moins un système de fermeture sont disposés sur un même emplacement de contenant.

Selon un autre mode de réalisation de l'installation le au moins un système d'ouverture, les moyens d'inoculation et le au moins un système de fermeture sont disposés sur des emplacements de contenants qui sont décalés.

### BRÈVE DESCRIPTION DES DESSINS

L'invention sera mieux comprise et d'autres caractéristiques et avantages apparaîtront plus clairement à la lecture de la description ci-après donnée à titre d'exemple et de manière non limitative, en référence aux dessins annexés dans lesquels :
- la figure 1 est un graphique présentant une courbe de mesure du nombre de cellules vivantes de micro-organismes en fonction du temps d'exposition ou de la dose de stérilisation, pour un procédé de stérilisation permettant d'établir une courbe de décroissance exponentielle ;
- la figure 2 est un graphique présentant la conversion logarithmique de cette courbe de décroissance ;
- la figure 3 est un graphique présentant une courbe de mesure du nombre de cellules vivantes de micro-organismes en fonction du temps d'exposition ou de la dose de stérilisation, pour un procédé de stérilisation ne donnant pas de courbe de décroissance exponentielle ;
- la figure 4 présente en coupe, suivant un plan vertical perpendiculaire au sens de défilement, le poste de travail d'une installation mettant en oeuvre un procédé de validation de stérilisation selon l'invention ;
- la figure 5 est une vue de dessus de cette installation ; et
- la figure 6 est une vue de dessus d'une installation suivant une variante.

### DESCRIPTION D'UN MODE DE RÉALISATION PRÉFÉRÉ DE L'INVENTION

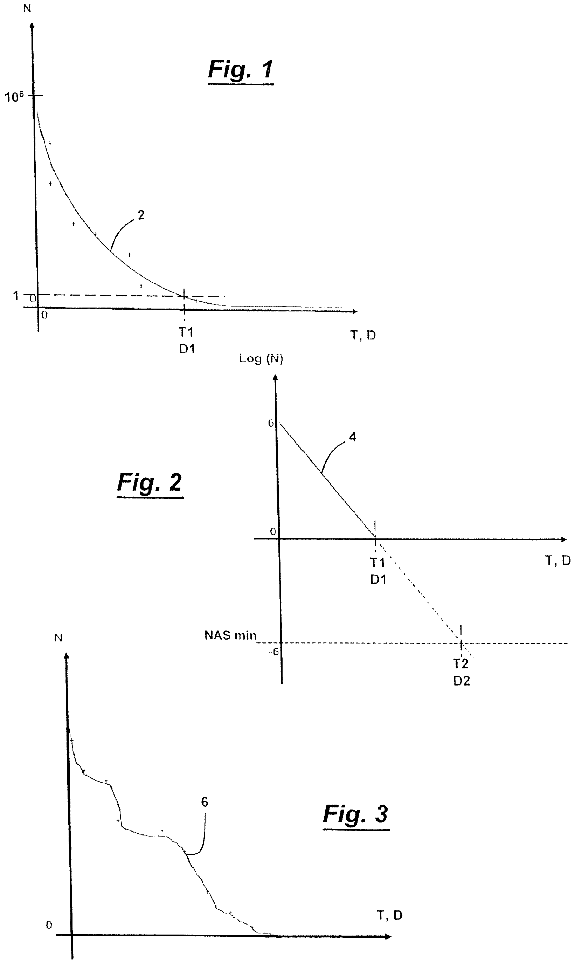

La figure 1 représente, pour un procédé de stérilisation de la première catégorie, une courbe 2 donnant le nombre N de cellules vivantes de micro-organismes mesuré après un temps d'exposition T du procédé ou après une dose D dans le cas de traitement par irradiation, après avoir introduit un indicateur biologique comportant une quantité N₀ de cellules vivantes de micro-organisme égale à 10⁶ dans un contenant fermé contenant un article.

On obtient une courbe exponentielle qui tend vers zéro pour un temps T assez long, ou pour une dose D assez forte.

En variante, on peut réaliser le même type de courbe pour un procédé de stérilisation en inoculant le contenant fermé avec une quantité N de cellules vivantes de micro-organismes inférieure à 10⁶, donnant de la même manière une courbe de décroissance exponentielle en fonction de la quantité inoculée au départ au sein de ce produit.

La figure 2 représente en fonction du temps d'exposition T, ou de la dose appliquée D, une droite 4 calculée pour les valeurs log (N) positives, par la conversion logarithmique log (N) du nombre N de cellules vivantes de micro-organismes présentée à la figure 1. La droite 4 démarre au temps T = 0 par la valeur log (N) = 6 correspondant à la contamination par 10⁶ cellules.

On notera avec log (1) = 0 que la conversion logarithmique d'une courbe exponentielle donne ainsi une droite qui passe par 0 au temps T1 (ou à la dose D1) correspondant au nombre N = 1 représentant une seule cellule vivante de micro-organisme mesurée.

Différentes études proposent, suivant une méthode de validation connue pour ces procédés de stérilisation, la possibilité d'extrapoler la droite dans les valeurs log (N) négatives en la continuant en dessous de 0. Ces valeurs négatives correspondent à un nombre N inférieur à 1, c'est-à-dire à une probabilité de moins d'une cellule de micro-organisme survivante par contenant traité.

En particulier, la norme européenne « NF EN556 » relative à la stérilisation de dispositifs médicaux demande une probabilité de présence d'une cellule de micro-organisme inférieure à 10⁻⁶, soit un risque maximum d'une chance sur un million qu'un article stérilisé contienne encore des micro-organismes survivants, ce qui est traduit par certains auteurs comme équivalent à la probabilité d'une cellule vivante de micro-organisme pour un million d'articles stérilisés. A partir d'une contamination log (N) = 6, il faut donc obtenir une réduction de 12 logs donnant la valeur log (N) = -6, laquelle valeur est atteinte en principe au temps de traitement nécessaire T2 ou à la dose D2.

Dans le cas d'un article comportant initialement un nombre plus fort ou plus faible de cellules vivantes de micro-organismes, la pente de la droite donnée par le procédé de stérilisation restant pour autant la même, il faut alors respectivement un temps d'exposition plus long ou plus court (ou une dose plus forte ou à l'inverse moindre) pour atteindre la valeur log (N) = -6.

En règle générale, les indicateurs biologiques couramment utilisés contiennent un nombre de cellules vivantes de micro-organisme compris entre 10⁶ et 10⁷.

Pour éviter de descendre dans des valeurs log (N) négatives, on pourrait partir d'un indicateur biologique présentant un niveau de contamination très élevé, par exemple égal à 12, pour atteindre la valeur finale égale à 0. Toutefois, ce type d'indicateur biologique n'est toujours pas disponible à ce jour sur le marché, du moins pour les micro-organismes de référence utilisés pour les procédés de stérilisation.

On notera que la contamination initiale moyenne d'un lot de dispositifs médicaux ou de produits injectables préparés pour la stérilisation est généralement faible, souvent inférieure à 10 cellules vivantes de micro-organismes. Toutefois, ce niveau de contamination peut être aléatoire, et l'on ne peut exclure une contamination initiale exceptionnelle qui, par exemple, s'approche du niveau maximal égal à 10⁶.

Un problème posé par cette méthode de validation est que, l'expérience montrant que certaines normes imposent un coefficient minimum de corrélation, il est parfois difficile d'établir la droite 4 de décroissance logarithmique. On ne peut alors pas établir les valeurs logarithmiques négatives, et l'on ne peut donc calculer le temps d'exposition nécessaire T2 ou la dose D2.

Un autre problème posé par cette méthode de validation est que la prolongation de la droite 4 vers les valeurs fortement négatives ne peut être expérimentalement vérifiée, et la marge d'erreur reste inconnue.

En particulier pour la valeur log (N) = -6, il faudrait tester un million d'unités dans des conditions identiques, ce qui n'est pas réalisable matériellement. On aurait notamment un problème de stabilité de l'indicateur biologique, qu'il faudrait placer dans tous les articles en un laps de temps très court afin d'éviter son évolution propre qui fausserait le test.

On ne peut en outre exclure que, pour les valeurs négatives de log (N), la loi d'évolution de la quantité de cellules de micro-organismes vivantes ne soit pas beaucoup plus complexe, les mécanismes de destruction de ces micro-organismes n'étant pas complètement élucidés à ce jour. Ces mécanismes dépendent notamment de la nature des micro-organismes, du procédé de stérilisation étudié, et des interactions entre le produit à stériliser et les types de micro-organismes, lesquelles sont souvent importantes.

La figure 3 représente, pour un procédé de stérilisation de la seconde catégorie, une courbe 6 donnant le nombre N de cellules vivantes de micro-organismes mesuré après un temps d'exposition T du procédé, après avoir introduit un indicateur biologique comportant une quantité de cellules vivantes de micro-organisme égale à 10⁶ dans un contenant fermé renfermant un article.

On obtient une courbe 6 descendant de manière continue, qui ne présente pas de forme particulière.

Les textes présentant ces procédés de stérilisation de la seconde catégorie prévoient d'inoculer les articles avec des indicateurs biologiques contenant un nombre défini de cellules vivantes de micro-organismes, souvent compris entre 10³ et 10⁶, puis de vérifier après la stérilisation que la totalité des indicateurs a été détruite.

Ne pouvant établir de courbe régulière de mesure du nombre de cellules à partir d'un paramètre, on ne peut donc pas calculer de probabilité de non stérilité pour ces procédés. D'ailleurs, dans certains cas, la réglementation ainsi que l'homme de l'art considèrent qu'il s'agit d'une décontamination sans obligation d'atteindre l'état de stérilité.

De plus, un autre problème posé par l'ensemble des méthodes de validation présentées ci-dessus, à l'exception notable de la méthode utilisant une très haute pression hydrostatique, est qu'on ne peut pas traiter une charge de stérilisation de manière homogène. En particulier, pour les procédés utilisant la chaleur sèche, la chaleur humide, ou encore l'oxyde d'éthylène, il existe systématiquement des écarts de température dans la charge, et pour les procédés réalisant une irradiation on obtient des écarts de dose entre différents points de la charge.

On a alors des variations importantes d'assurance de stérilité qui sont fréquemment estimées à plusieurs logs de réduction de la contamination.

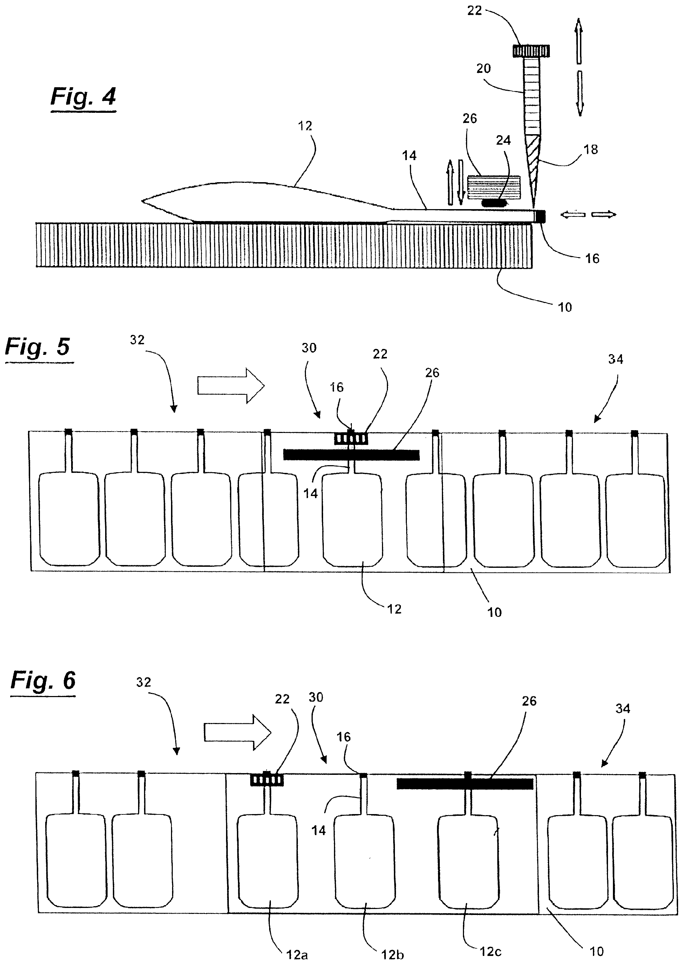

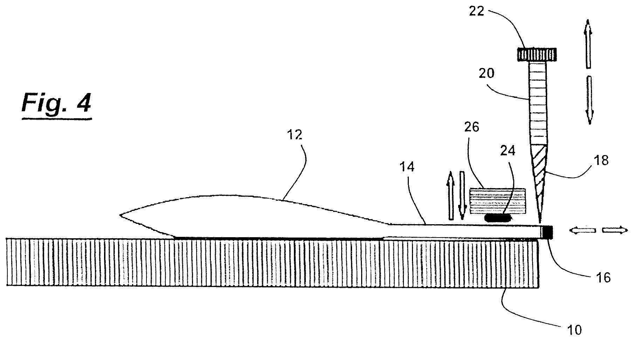

Les figures 4 et 5 présentent une installation apte à mettre en oeuvre la méthode de validation selon l'invention concernant un procédé de stérilisation quelconque, qui peut aussi bien appartenir à la première qu'à la seconde catégorie.

L'installation comporte un plateau 10 allongé recevant une succession de contenants 12 alignés dans la direction longitudinale, contenant chacun un produit à stériliser. Le contenant peut présenter des formes et des tailles variées. Le contenant peut être en particulier une poche, un sachet, un flacon rigide ou souple, une seringue, une ampoule rigide ou souple, une barquette thermoformée, ou tout autre contenant destiné à contenir tout produit à stériliser. Parmi ces produits à stériliser, nous pouvons citer notamment, mais pas exclusivement, les produits de santé, et notamment, les médicaments (chimiques ou biotechnologiques), les dispositifs médicaux, les produits cosmétiques, les produits de thérapie génique, les médicaments de thérapie innovante, les tissus et organes à greffer, les produits alimentaires, etc...

Pour plus de simplicité dans la description, l'exemple cité ci-dessous fera appel à un type de contenant traditionnel, à savoir la poche souple, contenant un produit liquide.

L'avance des poches 12 dans la direction longitudinale se fait pas à pas, au moyen d'un système manuel ou automatisé.

Les poches 12 ont subi au préalable un premier cycle de stérilisation, après une première contamination réalisée sur un ou plusieurs échantillons d'articles à stériliser, avec un nombre de cellules vivantes de micro-organismes UFC de 10⁶ utilisant préférentiellement un micro-organisme prouvé comme étant le plus difficile à stériliser par le procédé de stérilisation étudié.

Les poches 12 posées à plat, et maintenues sur le plateau 10 par un dispositif de bridage mécanique si nécessaire, présentent des cols 14 disposés parallèlement dans la direction transversale, cols qui sont fermés par un bouchon 16. Le plateau 10 comporte un moyen de translation longitudinale des poches 12 dans une direction d'avance, comme un convoyeur.

Le plateau 10 comporte un poste de travail central 30 qui se trouve entre des poches amont à traiter 32 et des poches aval déjà traitées 34. Le poste de travail 30 maintient la poche 12 en cours de traitement dans la même position pendant les opérations successives d'ouverture, de première ou de deuxième contamination, puis de fermeture de cette poche.

On notera qu'il est intéressant de réaliser les deux contaminations dans des conditions similaires, en particulier au même poste de travail maintenant la poche 12 dans la même position, avec un temps d'ouverture identique, afin de ne pas modifier les paramètres permettant d'obtenir des effets comparables pour ces inoculations.

Le plateau 10 avec ses équipements fixés dessus peut être disposé horizontalement, ou incliné dans la direction transversale, en particulier suivant un plan vertical, en relevant le col 14 afin d'éviter des fuites de produits lors de l'ouverture des poches 12.

Le plateau 10 supporte une lame de coupe 18 fixée sur un guidage vertical 20, coupant le col 14 transversalement au plus près de son bouchon 16 afin de modifier le moins possible le volume intérieur du contenant. La lame de coupe 18 peut être actionnée soit manuellement, en pressant sur un appui supérieur 22, soit automatiquement avec par exemple un actionneur électrique, comprenant un électroaimant.

Après l'ouverture de la poche 12 réalisée par la coupure de son col 14, le procédé comporte une étape de contamination qui peut être effectuée manuellement ou automatiquement, avec par exemple une seringue ou une pipette simple ou multiple dans le cas où plusieurs poches ont été ouvertes simultanément.

Le plateau 10 supporte un dispositif de soudage adapté pour chaque type de poche 12 après la coupe de son col 14, un tel dispositif comprenant une matrice 24 montée en dessous d'un système de descente 26 qui vient souder ce col au plus près de la coupe, et ce afin aussi de limiter la modification du volume intérieur et la perte de micro-organismes se trouvant dedans.

On peut réaliser en particulier une soudure par résistance à chauffage continu ou discontinu, par soudure haute fréquence, par un électroaimant de forte inductance ou encore par tout autre système adapté à la fermeture de la poche.

Le système de descente 26 peut être actionné manuellement ou automatiquement, avec par exemple un actionneur électrique à électroaimant. Le système de descente 26 est remonté après la soudure, pour libérer la poche 12 qui peut alors se déplacer dans la direction longitudinale.

Après si nécessaire une ouverture du dispositif de bridage mécanique de la poche 12 sur le plateau 10, l'ensemble des poches est déplacé longitudinalement pour disposer une nouvelle poche sur le poste de travail 30, et évacuer les poches en aval vers le stérilisateur avec une manutention qui peut être manuelle ou automatisée. Avantageusement, pour la deuxième stérilisation on replace les mêmes poches dans le stérilisateur au même endroit que pour la première, et ce afin d'obtenir des conditions de stérilisation identiques.

Après avoir disposé (pour le premier cycle) ou replacé (pour le deuxième cycle) toutes les poches 12 dans le stérilisateur, le cycle de stérilisation est lancé. Après le premier cycle, on réalise une révélation des indicateurs biologiques afin de vérifier qu'aucun indicateur n'a poussé, et on procède de même après le deuxième cycle.

L'absence de pousse à la suite du premier cycle permet de prouver que ce cycle a détruit au moins 10⁶ spores du micro-organisme le plus difficile à détruire par le procédé de stérilisation considéré ou, ce qui est équivalent, qu'il a réduit la contamination d'au moins 6 logs de ce micro-organisme.

L'absence de pousse à la suite du deuxième cycle permet de prouver de même que ce cycle réduit la contamination d'au moins 6 logs de ce même micro-organisme.

Avantageusement, l'homme de l'art peut réaliser un deuxième cycle différent du premier si ce deuxième cycle ne permet pas de détruire complètement les indicateurs biologiques inoculés, et donc ne permet pas de réduire la contamination d'au moins 6 logs. Cela peut s'expliquer notamment par des évolutions au niveau du produit ou du contenant dus au premier cycle de stérilisation.

Les deux cycles ayant été réalisés à la suite, l'addition des effets des deux cycles permet de prouver une réduction de la contamination de 6 + 6 = 12 logs de ce micro-organisme, comme indiqué par les courbes plus haut de la figure 2. Si la contamination initiale de l'article à stériliser est au plus égale à 10⁶ spores, le niveau d'assurance de stérilité NAS de 10⁻⁶ est atteint après 12 logs de réduction de contamination comme indiqué par la figure 2.

Un indicateur biologique est constitué du micro-organisme le plus difficile à détruire par le procédé de stérilisation considéré, et une contamination initiale égale à 10⁶ spores de ce micro-organisme correspond à un cas très défavorable. L'atteinte d'un niveau d'assurance de stérilité NAS de 10⁻⁶ pour une contamination initiale de 10⁶ spores d'indicateur biologique correspond à une situation décrite dans des textes de référence comme des conditions de sur-destruction. Les présents essais de validation décrits dans la méthode selon l'invention permettent donc de garantir l'atteinte d'un NAS de 10⁻⁶ dans tous les cas où la contamination microbienne de l'article à stériliser est maîtrisée à un niveau raisonnable, compatible avec celui décrit dans la bibliographie et les textes réglementaires, c'est-à-dire nettement inférieure à 10⁶ cellules vivantes de micro-organismes, pour un cocktail de micro-organismes moins résistants que les spores des indicateurs biologiques.

En routine, il suffit de reproduire à l'identique les conditions de validation composées d'un premier cycle de stérilisation, d'un temps d'attente dans les conditions décrites lors des essais de validation (temps, température, pression, etc...), et d'un deuxième cycle de stérilisation identique également à celui réalisé lors des essais de validation. L'ensemble de cette séquence permet de garantir de façon certaine et prouvée l'atteinte d'un NAS de 10⁻⁶ si la contamination initiale est inférieure ou égale à 10⁶ spores. L'ensemble de cette séquence permet donc de garantir de façon certaine et prouvée l'atteinte de ce NAS de 10⁻⁶ si la contamination initiale des produits à stériliser est inférieure à 10⁶ cellules vivantes de micro-organismes, quels que soient ces micro-organismes, puisqu'ils sont moins résistants au procédé de stérilisation que les spores.

En routine, les poches ne seront pas contaminées pour des raisons évidentes de sécurité sanitaire. Le cycle suivant sera utilisé : première stérilisation, temps d'attente correspondant au temps nécessaire pour l'ouverture, la deuxième inoculation et la fermeture des poches dans des conditions identiques à celles utilisées en validation (temps, température, pression, etc...), deuxième stérilisation.

La figure 6 présente un plateau 10 comportant un poste de travail central 30 recevant trois poches 12a, 12b, 12c décalées longitudinalement. On réalise simultanément sur la première poche 12a l'opération de coupe avec la lame de coupe 18, sur la deuxième poche 12b l'inoculation, et sur la troisième poche 12c la soudure du col.

Après cette série d'opérations simultanées, l'ensemble des poches 12 avance d'un pas dans la direction longitudinale, afin de recommencer les mêmes opérations sur les poches suivantes. On réalise de cette manière un gain de productivité pour l'installation, en particulier avec des opérations de coupe et de soudure automatisées, car les trois opérations sont réalisées en même temps.

Comme il va de soi, l'invention ne se limite pas aux seuls modes d'exécution préférentiels décrits ci-dessus.

Elle embrasse au contraire toutes les variantes possibles de réalisation, pour autant que ces dernières ne sortent pas du cadre délimité par les revendications ci-jointes qui définissent la portée de la présente invention.

Ainsi, les produits ou articles à stériliser peuvent être autres que des produits ou articles de santé, dès lors qu'il est conseillé qu'ils soient soumis à une stérilisation. De même, les contenants peuvent être autres que des poches dès lors que de tels contenants sont aptes à être ouverts par un système de découpe puis aussitôt après rescellés, le tout en un temps court.

En toute hypothèse, il convient bien sûr que l'installation décrite permette une inoculation et une fermeture rapide de l'ensemble des contenants d'une charge de stérilisation, et ce afin de minimiser le temps global qui s'écoule entre les deux cycles de stérilisation réalisés en validation.

## Revendications

1. Méthode de validation d'un procédé de stérilisation d'un article, en particulier un produit ou un dispositif destiné à la santé, permettant de valider le niveau d'assurance de stérilité atteint avec ce procédé de stérilisation, **caractérisé en ce qu'**elle consiste à réaliser une première étape de contamination d'un contenant (12) recevant l'article par plus de 10⁵ cellules vivantes de micro-organisme, à réaliser ensuite un premier cycle de stérilisation avec le procédé retenu, à ouvrir ensuite le contenant (12) pour le contaminer à nouveau par plus de 10⁵ cellules vivantes de micro-organisme, puis à réaliser un deuxième cycle de stérilisation avec le même procédé, et enfin à vérifier la stérilité du contenant (12) après le premier cycle et après le deuxième cycle de stérilisation.

2. Méthode de validation selon la revendication 1, **caractérisée en ce que** chaque contamination est effectuée avec au moins 10⁶ cellules vivantes de micro-organisme.

3. Méthode de validation selon la revendication 1 ou 2, **caractérisée en ce que** les contaminations sont faites avec les cellules vivantes du micro-organisme prouvé comme étant le plus difficile à éliminer avec le procédé de stérilisation retenu.

4. Méthode de validation selon l'une quelconque des revendications 1 à 3, **caractérisée en ce que** la vérification de la stérilité du contenant (12) comporte la révélation d'indicateurs biologiques démontrant qu'aucun de ces indicateurs n'a réalisé une croissance.

5. Méthode de validation selon l'une quelconque des revendications 1 à 4, **caractérisée en ce que** les deux contaminations sont effectuées dans les mêmes conditions.

6. Méthode de validation selon l'une quelconque des revendications 1 à 5, **caractérisée en ce que** les deux cycles de stérilisation sont effectués dans les mêmes conditions.

7. Méthode de validation selon l'une quelconque des revendications 1 à 6, **caractérisée en ce que** le cycle de routine est composé d'un premier cycle de stérilisation, d'un temps d'attente et d'un deuxième cycle de stérilisation.

8. Méthode de validation selon la revendication 7, **caractérisée en ce que** le temps d'attente des cycles de routine compris entre les deux cycles de stérilisation est égal au temps nécessaire pour réaliser la contamination de la charge entre le premier et le deuxième cycle lors de la validation.

9. Méthode de validation selon l'une quelconque des revendications 7 et 8, **caractérisée en ce que** les conditions d'environnement (temps, température et pression notamment) des cycles de routine sont identiques ou le plus proche possible des conditions d'environnement lors de la contamination de la charge entre le premier et le deuxième cycle.

## Patentansprüche

1. Validierungsmethode eines Sterilisierungsverfahrens für einen Gegenstand, insbesondere ein Produkts oder eine Vorrichtung für die Gesundheit, welche es ermöglicht, das Sicherheitsniveau der Sterilität zu validieren, das mit diesem Sterilisierungsverfahren erreicht wurde, **dadurch gekennzeichnet, dass** die Methode darin besteht, einen ersten Kontaminationsschritt eines Behälters (12) zu verwirklichen, der den Gegenstand mit über 10⁵ lebenden Zellen von Mikroorganismen aufnimmt, danach einen ersten Sterilisationszyklus mit dem in Betracht gezogenen Verfahren zu verwirklichen, danach den Behälter (12) zu öffnen, um ihn erneut mit mehr als 10⁵ lebenden Zellen von Mikroorganismen zu kontaminieren, dann einen zweiten Sterilisationszyklus mit dem selben Verfahren zu verwirklichen, und schließlich die Sterilität des Behälters (12) nach dem ersten Zyklus und nach dem zweiten Zyklus der Sterilisation zu verifizieren.

2. Validierungsmethode nach Anspruch 1, **dadurch gekennzeichnet, dass** jede Kontamination mit wenigstens 10⁶ lebenden Zellen von Mikroorganismen durchgeführt wird.

3. Validierungsmethode nach Anspruch 1 oder 2, **dadurch gekennzeichnet, dass** die Kontaminationen mit den lebenden Zellen von Mikroorganismen durchgeführt werden, die sich als die mit dem in Betracht gezogenen Sterilisierungsverfahren am schwierigsten zu eliminieren erwiesen haben.

4. Validierungsmethode nach einem der Ansprüche 1 bis 3, **dadurch gekennzeichnet, dass** die Verifizierung der Sterilität des Behälters (12) die Erfassung von biologischen Indikatoren umfasst, die aufzeigt, dass keiner dieser Indikatoren ein Wachstum erfahren hat.

5. Validierungsmethode nach einem der Ansprüche 1 bis 4, **dadurch gekennzeichnet, dass** die beiden Kontaminationen unter denselben Bedingungen durchgeführt werden.

6. Validierungsmethode nach einem der Ansprüche 1 bis 5, **dadurch gekennzeichnet, dass** die beiden Sterilisationszyklen unter denselben Bedingungen durchgeführt werden.

7. Validierungsmethode nach einem der Ansprüche 1 bis 6, **dadurch gekennzeichnet, dass** der Routinezyklus gebildet wird aus einem ersten Sterilisationszyklus, einer Wartezeit und einem zweiten Sterilisationszyklus.

8. Validierungsmethode nach Anspruch 7, **dadurch gekennzeichnet**, das die Wartezeit des Routinezyklus zwischen den beiden Sterilisationszyklen gleich der Zeit ist, die notwendig ist, um die Kontamination der Charge zwischen dem ersten Zyklus und dem zweiten Zyklus bei der Validierung zu verwirklichen.

9. Validierungsmethode nach einem der Ansprüche 7 oder 8, **dadurch gekennzeichnet, dass** die Umgebungsbedingungen (Zeit, Temperatur und Druck vor allem) der Routinezyklen den Umgebungsbedingungen bei der Kontamination der Charge zwischen dem ersten und dem zweiten Zyklus identisch oder so weit wie möglich angenähert sind.

## Claims

1. Method for validating a sterilisation process of an item, in particular a product or a device intended for healthcare, allowing one to validate the level of assurance of the sterility reached with said sterilisation process, **characterised in that** it consists in carrying out a first step of contaminating a container (12) receiving the item with more than 10⁵ living cells from micro-organisms, then carrying out a first sterilisation cycle using the selected process, then opening the container (12) to contaminate it again with more than 105 living cells from micro-organisms, then carrying out a second sterilisation cycle using the same process and, finally, in verifying the sterility of the container (12) after the first cycle and after the second cycle of sterilisation.

2. Validation method according to claim 1, **characterised in that** each contamination is carried out with at least 10⁶ living cells from micro-organisms.

3. Validation method according to claim 1 or 2, **characterised in that** the contaminations are carried out using living cells from the micro-organism that has been proven to be the most difficult to eliminate by using the sterilisation process selected.

4. Validation method according to any of claims 1 to 3, **characterised in that** the verification of the sterility of the container (12) comprises the revelation of biological indicators demonstrating that none of these indicators has increased.

5. Validation method according to any of claims 1 to 4, **characterised in that** the two contaminations are carried out under the same conditions.

6. Validation method according to any of claims 1 to 5, **characterised in that** the two sterilisation cycles are carried out under the same conditions.

7. Validation method according to any of claims 1 to 6, **characterised in that** the routine cycle comprises a first sterilisation cycle, a waiting time and a second sterilisation cycle.

8. Validation method according to claim 7, **characterised in that** the waiting time for the routine cycles comprised between the two sterilisation cycles equals the time required to contaminate the load between the first and second cycles during validation.

9. Validation method according to any of claims 7 and 8, **characterised in that** the environment conditions (time, temperature and pressure, in particular) for the routine cycles are identical to or as close as possible to the environment conditions during the contamination of the load between the first and second cycles.
